# EUROPEAN PATENT APPLICATION

(11) **EP 4 650 454 A1**
(43) Date of publication of application: **19.11.2025**
(21) Application number: 23916204.3
(22) Date of filing: 14.12.2023
(51) Int. Cl.: C12P 1/02, C12N 1/00, C12N 1/14, C12P 7/00

(54) **METHOD FOR EXTRACTING MYCOSPORINE-LIKE AMINO ACIDS**

(30) Priority: 10.01.2023 JP 2023002010
(71) Applicant: Nissui Corporation, Tokyo 105-8676 (JP)
(72) Inventor: NAKAJIMA, Toshiaki, Hachioji-shi, Tokyo 192-0991 (JP)
(74) Representative: Mewburn Ellis LLP
(86) International application number: PCT/JP2023/044828
(87) International publication number: WO 2024/150590

(57) **Abstract**

An object of the present disclosure is to efficiently extract mycosporine-like amino acids (MAAs), and to obtain an MAA extract at high concentration, from fungal cells of a black yeast of the family Dothioraceae of the order Dothideales. The present disclosure provides a method of extracting MAAs into water, the method including: a culture step of culturing *Aureobasidium pullulans;* and an extraction step of extracting an MAA fraction from cultured fungal cells of the *Aureobasidium pullulans*; the method satisfying one or more selected from the following (a), (b), and (c): (a) in the extraction step, an MAA fraction is extracted into a polar solvent at not less than 40°C; (b) before the extraction step, a heating step of collecting fungal cells from the culture liquid after the culture step, and heating the collected fungal cells, is carried out; (c) in the culture step, the culture is carried out in a medium that does not substantially contain ascorbic acid or a salt thereof, and/or peptone.

## Description

### TECHNICAL FIELD

The present disclosure relates to a method of extracting mycosporine-like amino acids (hereinafter also referred to as MAAs) from fungal cells into a polar solvent, especially water, and also relates to a fungal cell extract containing MAAs.

### BACKGROUND ART

MAAs are naturally occurring substances known to efficiently absorb ultraviolet rays such as UV-A and UV-B. MAAs are expected to be applied as a UV-protective agent to be included in sunscreen cosmetics and the like. Aquatic organisms such as algae and shellfish, as well as microorganisms such as yeasts and molds, are known to produce MAAs, and several ten kinds of such organisms have been found.

Attempts have been made to extract and obtain MAAs from various organisms.

It has been reported that MGGnol (mycosporine-glutaminol-glucoside) and MGGcol (mycosporine-glutamicol-glucoside), which are MAAs, can be extracted from lyophilized fungal cells of yeasts and fungi using aqueous methanol at low temperature (Non-Patent Documents 1 to 3).

Among the species classified in the family Dothioraceae of the order Dothideales, a group of fungi that are generally called black yeasts because of their black fungal cells are also known to produce MAAs. For example, *Aureobasidium pullulans* and *Hortaea werneckii* are also known to produce MGGnol and MGGcol (Non-Patent Document 2), and Patent Document 1 discloses that their extraction from cultured fungal cells (lyophilized fungal cells) is possible using 100% water or 1 to 20% aqueous ethanol solution at normal temperature, and that more efficient extraction is possible when the aqueous ethanol solution is used.

There are also cases where extraction was carried out under high-temperature conditions. For example, it has been disclosed that MGGnol was extracted using 20% aqueous methanol solution at 80°C for 4 hours from lyophilized fungal cells of *Cryptococcus stepposus* (Non-Patent Document 4).

However, for MAAs that have been treated under high temperature conditions, there has been a concern about their stability. Mycosporine, mycosporine-glycine, and porphyra-334 have been reported to undergo degradation at 75 to 80°C (Non-Patent Documents 5 and 6).

### PRIOR ART DOCUMENTS

### [Patent Document]

[Patent Document 1] JP 5913988 B

### [Non-patent Documents]

[Non-Patent Document 1] M. Volkmann et al., FEMS Microbiol. Lett., 255, 286-295 (2006)
[Non-Patent Document 2] T. Kogej et al., Environ. Chem., 3, 105-110 (2006)
[Non-Patent Document 3] C. F. Chang et al., Arch. Microbiol., 201, 27-33 (2019)
[Non-Patent Document 4] M. Moline, et al., RADIATION RESEARCH 175, 44-50 (2011)
[Non-Patent Document 5] N. Wada et al., Antioxidants, 4, 603-646 (2015)
[Non-Patent Document 6] M. Yoshiki et al., Food Chemistry, 113, 1127-1132 (2009)
[Non-Patent Document 7] N.A. Yurlava et al., Studies in Mycology.,43,63-69(1999)
[Non-Patent Document 8] de Hoog et al., Atlas of clinical fungi, 2nd edition: Utrecht: centraalubureauvoor Schimmelcultures; (2000)
[Non-Patent Document 9] G. S. de Hoog et al., Antonie van Leeuwenhoek., 65, 41-54 (1994)

### SUMMARY OF THE INVENTION

### PROBLEMS TO BE SOLVED BY THE INVENTION

From the viewpoint of the fact that the extracted MAAs are added to a composition such as a cosmetic, and the fact that removal of the solvent from the extract is laborious, there has been a demand for avoiding the use of an organic solvent as the solvent for the extraction of MAAs from fungal cells of a MAA-producing fungus. However, the extraction efficiency is not necessarily high in cases where the extraction is carried out using only water at normal temperature. The low extraction efficiency leads to a low concentration of the extract, which may result in a laborious and/or costly process when the MAAs are provided for various uses.

If a culture liquid of the fungal cells can be directly subjected to an extraction step, or if sterilization treatment can be carried out after inclusion of the extracted MAAs, the labor and/or the cost can be reduced, which is preferred. However, such a method is not known to the best of the inventors' knowledge.

An object of the present disclosure is to efficiently extract MAAs from fungal cells of a black yeast of the family Dothioraceae of the order Dothideales, and to obtain an MAA extract at high concentration.

### MEANS FOR SOLVING THE PROBLEMS

In order to solve the above problem, the inventors of the present disclosure intensively studied to infer that MAAs can be efficiently extracted by providing: a step of heating fungal cells of a black yeast of the family Dothioraceae of the order Dothideales, or heating a culture liquid containing such cells; and/or a culture step of performing culture using a specific medium; before an extraction step.

Specifically, the present disclosure includes the following inventions. The term "MAAs" may include a single type or a plurality of types of mycosporine-like amino acids.
[1] A polar solvent extract of cultured fungal cells of a black yeast of the family Dothioraceae of the order Dothideales, the extract comprising a mycosporine-like amino acid (MAA), wherein the MAA yield per fungal cell culture liquid is not less than 0.06 g/L.
[2] The polar solvent extract according to [1], wherein the MAA comprises mycosporine-glutaminol-glucoside (MGGnol) and/or mycosporine-glutamicol-glucoside (MGGcol).
[3] The extract according to [1] or [2], wherein the polar solvent is water.
[4] The extract according to any one of [1] to [3], wherein the black yeast is a black yeast belonging to the genus *Aureobasidium,* the genus *Hormonema (Sydowia),* or the genus *Hortaea.*
[5] A method of extracting an MAA into a polar solvent, the method comprising:
   a culture step of culturing a black yeast of the family Dothioraceae of the order Dothideales; and
   an extraction step of extracting an MAA fraction from cultured fungal cells of the black yeast;
   the method satisfying one or more selected from the following (a), (b), and (c):
      (a) in the extraction step, the MAA fraction is extracted into a polar solvent at not less than 40°C;
      (b) before the extraction step, a heating step of collecting fungal cells from the culture liquid after the culture step, and heating the collected fungal cells, is carried out; and
      (c) in the culture step, the culture is carried out in a medium that does not substantially contain ascorbic acid or a salt thereof, and/or peptone.
[6] The method according to [5], wherein the (a) is carried out by heating the culture liquid after the culture step to not less than 40°C.
[7] The method according to [5] or [6], wherein the (b) heating step is carried out by heating under one or more conditions selected from the group consisting of (i) to (iii):
   (i) a temperature of not less than 105°C;
   (ii) a heating time of not less than 2 hours; and
   (iii) a pressure of not more than 0.10 MPa.
[8] The method according to any one of [5] to [7], wherein the MAA comprises MGGnol and/or MGGcol.
[9] The method according to any one of [5] to [8], wherein the polar solvent is water.
[10] The method according to any one of [5] to [9], wherein ethanol and/or butyl alcohol is/are not substantially used as the extraction solvent.
[11] The method according to any one of [5] to [10], wherein the black yeast is a black yeast belonging to the genus *Aureobasidium,* the genus *Hormonema (Sydowia),* or the genus *Hortaea.*
[12] A method of extracting an MAA, the method comprising:
   a culture step of culturing a black yeast of the family Dothioraceae of the order Dothideales; and
   an extraction step of extracting an MAA fraction from cultured fungal cells of the black yeast into a polar solvent;
   wherein the survival rate of the fungal cells after the extraction step is not more than 20% compared to the fungal cells after the culture step.
[13] The method according to [12], comprising, during or before the extraction step, exposing the cultured fungal cells and/or the culture liquid after the culture step to not less than 40°C.
[14] The method according to [12] or [13], wherein the polar solvent is water.
[15] The method according to any one of [12] to [14], wherein the black yeast is a black yeast belonging to the genus *Aureobasidium,* the genus *Hormonema (Sydowia),* or the genus *Hortaea.*
[16] A method for producing a polar solvent extract of cultured fungal cells of a black yeast of the family Dothioraceae of the order Dothideales, the method comprising extracting an MAA by the method according to any one of [5] to [15].
[17] A medium for culturing a black yeast of the family Dothioraceae of the order Dothideales, the medium not substantially comprising ascorbic acid or a salt thereof, and/or peptone.
[18] The medium according to [17], wherein the black yeast is a black yeast belonging to the genus *Aureobasidium,* the genus *Hormonema (Sydowia),* or the genus *Hortaea.*
[19] A method of producing an MAA-containing composition, the method comprising a heating step of heating an MAA-containing composition.
[20] The production method according to [19], wherein the heating step is carried out at a temperature of not less than 40°C.
[21] The production method according to [19] or [20], further comprising a pressurization step of pressurizing the MAA-containing composition.
[22] The production method according to any one of [19] to [21], wherein the MAA-containing composition comprises a cultured fungal cell extract of a black yeast of the family Dothioraceae of the order Dothideales.
[23] The production method according to any one of [19] to [22], comprising:
   a culture step of culturing a black yeast of the family Dothioraceae of the order Dothideales;
   an extraction step of extracting an MAA fraction from cultured fungal cells of the black yeast; and
   a step of including the MAA fraction into the composition;
   before the heating step.

### EFFECT OF THE INVENTION

The present disclosure enables extraction of MAAs from fungal cells of a black yeast of the family Dothioraceae of the order Dothideales with a high recovery. This improved amount of MAAs recovered is thought to be achieved due to the fact that heating of the fungal cells of the black yeast of the family Dothioraceae of the order Dothideales promotes release of MAAs to the outside of the fungal cells, and the fact that inactivation of degrading enzymes in the fungal cells occurs to suppress degradation of MAAs. In addition, since the amount of MAAs produced by the fungal cells can be increased by performing culture in a specific medium, the amount of MAAs recovered can be further improved. The increased recovery of MAAs allows production of a polar solvent extract, especially a water extract, containing MAAs at high concentration.

Further, the present disclosure enables extraction of MAAs within a shorter time than a conventional extraction method that uses water and/or aqueous ethanol at normal temperature.

Further, since water alone may be used as the extraction solvent in one mode of the present disclosure, MAAs can be advantageously obtained without using an organic-solvent-tolerant extraction device, and without carrying out post-treatment for removing an organic solvent.

Further, as shown by the present disclosure, MAAs are stable and not degraded by heating and/or pressurization, so a product containing MAAs can be subjected to sterilization in any step, which is industrially very advantageous.

### MODE FOR CARRYING OUT THE INVENTION

The black yeast of the family Dothioraceae of the order Dothideales is not limited, and any black yeast may be used as long as it produces MAAs. In general, yeasts whose fungal cells are black are called black yeasts, and the black yeasts include not only those classified in the family Dothioraceae of the order Dothideales, but also those belonging to the genus *Moniliella* of the phylum Basidiomycota, or those belonging to the genus *Exophiala* of the family Ferpotrichiellaceae of the order Chaetothyriales (Non-Patent Document 8). However, the black yeast used in the present disclosure may be a black yeast classified in the family Dothioraceae of the order Dothideales. More specifically, the present disclosure may use a black yeast belonging to the genus *Aureobasidium,* the genus *Hormonema,* or the genus *Hortaea.*

Here, the black yeast belonging to the genus *Hormonema* may include a black yeast whose teleomorph corresponds to the genus *Sydowia.* The anamorph of the genus *Sydowia* corresponds to the genus *Hormonema,* and the same fungal strain shows such a difference depending on whether its reproduction occurs sexually or asexually (Non-Patent Documents 7 and 8). In some cases, fungal cells described as the genus *Sydowia* in the present description may exhibit a yeast-like shape when they are grown by liquid culture, and can be judged as the genus *Hormonema,* which is the anamorph. Therefore, both are described in an inclusive manner (Non-Patent Document 9)

Further, although the fungi whose anamorphs belong to the genus *Aureobasidium* and the fungi whose anamorphs belong to the genus *Hormonema* have a close relationship to each other, these are clearly distinguishable by molecular phylogeny (Non-Patent Document 7).

In the present disclosure, the black yeast of the family Dothioraceae of the order Dothideales is not limited, and any strain may be used as long as it produces MAAs.

Examples of the black yeast of the genus *Aureobasidium* include *Aureobasidium pullulans, Aureobasidium subglaciale, Aureobasidium aerium, Aureobasidium microstictum,* and *Aureobasidium proteae.* Specific examples of their fungal strains include the NRBC 6353 strain, the NRBC 100716 strain, the NRBC 106987 strain, the NRBC 4464 strain, the NRBC 7757 strain, NRBC 108784, and the NRBC 114573 strain of *Aureobasidium pullulans* (all of these are available from the Patent Microorganisms Depositary, National Institute of Technology and Evaluation (NPMD) (Address: Room 122, 2-5-8 Kazusakamatari, Kisarazu-shi, Chiba, 292-0818 Japan). The IDF-23 strain (NPMD deposited strain, accession No. FERM P-22141), described in Patent Document 1, may also be used.

Examples of the black yeast of the genus *Hormonema* include *Hormonema macrosporum and Hormonema dematioides.* Specific examples of their fungal strains include the NRBC 107026 strain, the NRBC 107027 strain, the NRBC 107028 strain, the NRBC 5982 strain, and the NRBC 32065 strain (all of these are available from NPMD), which are distributed as *Sydowia polyspora* (teleomorph), whose anamorph corresponds to *Hormonema macrosporum.*

Examples of the black yeast of the genus *Hortaea* include *Hortaea werneckii.* Specific examples of its fungal strains include the NRBC 4875 strain and the NRBC 6407 strain of *Hortaea werneckii* (both of these are available from NPMD).

The black yeast of the family Dothioraceae of the order Dothideales to be subjected to the culture may be of either a single fungal strain or a plurality of fungal strains.

The medium used in the culture step of culturing a black yeast of the family Dothioraceae of the order Dothideales is not limited as long as the medium sufficiently allows the growth of the fungal strain, and may contain a carbon source, a nitrogen source, an inorganic salt, and, when necessary, components such as amino acids suitable for the growth of the microorganism. Examples of the carbon sources that may be used include sugars such as glucose, starch, and waste molasses; organic acids; and/or sodium acetate; especially sugars such as glucose. Examples of the nitrogen sources that may be used include ammonium salts, yeast extract, corn steep liquor, and peptone. Examples of the inorganic salts that may be used include magnesium salts, calcium salts, phosphate salts, iron salts, and copper salts. Usually, a liquid medium prepared by mixing and dissolving these components in water may be used.

The pH may usually be within the range of 2 to 9, 3 to 8, or 3.5 to 7.5. The growth of the fungus may be inhibited in cases where the pH is too high or too low.

The medium used may be, but does not necessarily need to be, a medium in a state where it does not substantially contain sodium ascorbate and/or peptone. The medium that "does not substantially contain" sodium ascorbate and/or peptone, used herein may be a medium in which the content of sodium ascorbate and/or peptone is not more than 0.1% by weight, not more than 0.01% by weight, or not more than 0.001% by weight, or is 0% by weight.

As shown in Examples below, the amount of MAAs produced increases in a black yeast of the family Dothioraceae of the order Dothideales cultured in a medium that does not substantially contain sodium ascorbate and/or peptone. Therefore, the amount of MAAs extracted from fungal cells of the yeast can be improved. Thus, the present description can also provide a medium that does not substantially contain sodium ascorbate and/or peptone, as a medium that can be suitably used for the culture of a black yeast of the family Dothioraceae of the order Dothideales.

The composition of the medium that does not substantially contain sodium ascorbate and/or peptone is not limited. Examples of the composition include a composition prepared by mixing glucose and yeast extract in water.

In the culture step, the culture temperature is not limited as long as it is a condition that sufficiently allows the growth of the fungal cells. The culture temperature may be, for example, 15 to 40°C, 18 to 35°C, or 20 to 30°C. In cases where the culture temperature is too low, the growth rate is insufficient, while in cases where the culture temperature is too high, the biosynthetic pathways tend to be abnormal.

In the culture step, the culture period is not limited. The culture may be carried out for 72 hours to 240 hours, or 96 hours to 168 hours.

Further, in the culture step, light irradiation may or may not be carried out. The culture cost can be reduced in cases where light irradiation is not carried out.

After the culture step, an extraction step of extracting an MAA fraction from the cultured fungal cells of the black yeast of the family Dothioraceae of the order Dothideales into a polar solvent is carried out.

Here, in the extraction step, the efficiency of extraction tends to be improved when the following (a) and/or (b) is/are satisfied. However, in cases where the following (c) is satisfied in the culture step, efficient extraction is possible even without carrying out a step satisfying (a) or (b). In other words, the extraction method of the present disclosure may satisfy one or more selected from the following (a), (b), and (c).
(a) In the extraction step, an MAA fraction is extracted into a polar solvent at not less than 40°C.
(b) Before the extraction step, a heating step of collecting fungal cells from the culture liquid after the culture step, and heating the collected fungal cells, is carried out.
(c) In the culture step, the culture is carried out in a medium that does not substantially contain ascorbic acid or a salt thereof, and/or peptone.

After the culture step, but before the extraction step, fungal cells may be collected from the culture liquid. The collection of the fungal cells may be carried out by centrifugation, filtration, or the like. The collected fungal cells may be dried using a heat dryer, a freeze dryer, a vacuum dryer, a flash drier, or the like.

In the method of the present disclosure, in the (a) extraction of the MAA fraction into the polar solvent at not less than 40°C, the fungal cells are brought into contact with the extraction solvent.

As the extraction solvent, a polar solvent may be used, or a polar solvent containing mainly water may be used. Examples of such a polar solvent include water; and polar organic solvents such as lower alcohols (including methanol, ethanol, butyl alcohol, isopropanol, and butylene glycol) and acetone. Examples of the polar solvent containing mainly water include water; and aqueous solutions of a polar organic solvent such as a lower alcohol (for example, methanol, ethanol, butyl alcohol, isopropanol, or butylene glycol) or acetone. Water alone may also be used. As the solvent "containing mainly water", a solvent containing water at not less than 80% by weight, not less than 85% by weight, or not less than 90% by weight in the total extraction solvent may be used. The higher the water content in the extraction solvent, the higher the efficiency of extraction can be expected to be. In other words, in cases where an aqueous solution of a polar organic solvent such as a lower alcohol is used as the extraction solvent, the concentration of the polar organic solvent may be not more than 20% by weight, or the organic solvent may not be substantially used. Here, "the organic solvent is not substantially used" means that the concentration of the polar organic solvent in the aqueous solution used as the extraction solvent is not more than 1% by weight, not more than 0.1% by weight, or not more than 0.01% by weight. In cases where the polar organic solvent is not substantially used in the extraction, the step of evaporating the polar organic solvent from the obtained extract can be eliminated.

The temperature of the extraction solvent in the extraction of the MAA fraction is 40°C to 95°C, or may be 50°C to 90°C, or 60°C to 85°C. In cases where the temperature of the extraction solvent is too low, the extraction efficiency tends to decrease. In cases where the temperature of the extraction solvent is too high, a problem tends to occur in the stability of a desired component. Further, an apparatus such as an autoclave may be used to perform heating to not less than 90°C, or to a temperature of 100°C to 135°C, 110°C to 130°C, or 120°C to 125°C. The heating can inhibit the actions of undesired enzymes contained in the cells. In cases where the heating temperature is too low, the inhibition of the actions of undesired enzymes may be insufficient, while in cases where the heating temperature is too high, a problem tends to occur in the stability of a desired component. Further, pressurization may be carried out to not less than 0.10 MPa, not less than 0.12 MPa, not less than 0.15 MPa, or not less than 0.20 MPa. The pressurization promotes the inhibition of the actions of undesired enzymes contained in the cells. In cases where the pressurization is insufficient, the inhibition of the actions of undesired enzymes contained in the cells may be insufficient. From the viewpoint of safety, the pressurization may be carried out at not more than 1.00 MPa.

The extraction time may be 15 minutes to 120 minutes, 60 minutes to 110 minutes, or 90 minutes to 100 minutes. The longer the extraction time, the more likely the extracted amount is to be increased. The upper limit of the extraction time may be, for example, about 120 minutes. In cases where the extraction time is too long, undesired impurities are likely to be contained. During the extraction step, the fungal cells and the extraction solvent that have been brought into contact with each other may be stirred or shaken as appropriate, or may be left to stand.

After the culture step, the culture liquid may be subjected, directly or after being diluted as appropriate, to the extraction step without collecting the fungal cells from the culture liquid, to carry out the (a) extraction of the MAA fraction into the polar solvent at not less than 40°C. In this case, the culture liquid may be heated to not less than 40°C or not less than 50°C. Efficient extraction can be expected by the heating of the culture liquid in advance. Usually, the culture liquid may contain a polar solvent, especially water.

The culture liquid after the culture step may be diluted, for example, not less than 2-fold by volume with water, a buffer, a medium, or the like before being subjected to the extraction step. In cases where the culture liquid is diluted with water, a buffer, a medium, or the like before its use in the extraction step, the culture liquid can be more easily handled. For example, in cases where the viscosity of the culture liquid is high, the culture liquid may be diluted with the buffer, medium, or the like before its use in the extraction step. By this, retention of the culture liquid in the apparatus can be reduced in some cases. The culture liquid may also be used directly in the extraction step. By the direct use of the culture liquid, the extraction cost may be reduced.

In the method of the present disclosure, the (b) heating step of heating, before the extraction step, the fungal cells collected from the culture liquid after the culture step may be carried out instead of or prior to the (a). In this step, under the following conditions, the fungal cells can be damaged or broken to improve the extraction efficiency of MAAs, or the actions of undesired enzymes in the fungal cells can be inhibited to improve the amount of MAAs recovered.

The heating of the fungal cells in the heating step may be carried out under conditions selected from the following in terms of the (i) temperature, (ii) length of time, and (iii) pressure.
(i) The temperature to which the fungal cells are exposed may be 40°C to 200°C, 50°C to 190°C, 60°C to 180°C, 70°C to 170°C, 80°C to 160°C, 90°C to 150°C, 100°C to 140°C, 105°C to 130°C, 110°C to 130°C, 115°C to 130°C, 120°C to 130°C, or 125°C to 130°C. In cases where the temperature to which the fungal cells are exposed is too low, the inhibition of the actions of undesired enzymes may be insufficient, while in cases where the temperature to which the fungal cells are exposed is too high, a problem tends to occur in the stability of a desired component.
(ii) The length of time for which the fungal cells are heated may be 2 to 6 hours, 3 to 5 hours, or 4 to 5 hours. The heating time may be a time for which a certain heating temperature is maintained after the temperature is achieved. In cases where the length of time for which the fungal cells are heated is too short, the inhibition of the actions of undesired enzymes may be insufficient, while in cases where the length of time for which the fungal cells are heated is too long, a problem tends to occur in the stability of a desired component.
(iii) The pressure applied to the fungal cells, calculated as the average pressure during the period when the fungal cells are exposed to heating, may be 0.01 MPa to 0.10 MPa, 0.02 MPa to 0.08 MPa, or 0.03 MPa to 0.05 MPa. In cases where the pressure applied to the fungal cells is too low, the inhibition of the actions of undesired enzymes may be insufficient, while in cases where the pressure applied to the fungal cells is too high, a problem tends to occur in the stability of a desired component.

The conditions (i) to (iii) may be arbitrarily selected and combined. In particular, at least (i) and (ii) may be combined. In this case, by heating at a high temperature for a certain length of time, sterilization can be achieved at the same time so that enzymes in the fungal cells can be inactivated. Therefore, the amount of MAAs recovered can be expected to be further improved. In addition, since the fungal cells are broken by (iii), the extraction efficiency of MAAs may be further improved. The heating step may be carried out under conditions where the fungal cells are dried. For example, the heating step may be carried out under one or more conditions selected from (i) a temperature of not less than 105°C, (ii) a heating time of not less than 2 hours, and (iii) a pressure of not more than 0.10 MPa. The heating step may also be carried out under the conditions of (i) a temperature of not less than 105°C, (ii) a heating time of not less than 2 hours, and (iii) a pressure of not more than 0.10 MPa.

In the method of the present disclosure, in cases where the extraction step is carried out using fungal cells that have been subjected to the (b) heating step, the extraction step may be carried out under the conditions described in (a), or may be carried out at a low extraction temperature.

Specifically, the extraction solvent used may be the same as that described in (a). On the other hand, the temperature of the extraction solvent during the extraction of the MAA fraction into the polar solvent may be not less than 5°C, may be not less than 10°C, or may be not less than 25°C (normal temperature). The conditions described in (a) are also applicable. Thus, the temperature may be 40°C to 70°C, 50°C to 90°C, or 60°C to 100°C, or an apparatus such as an autoclave may be used to perform heating to a temperature of 90°C to 150°C, 100°C to 140°C, 110°C to 130°C, or 120°C to 125°C.

The extraction time may be not less than 15 minutes, not less than 60 minutes, or not less than 90 minutes. The longer the extraction time, the more likely the extracted amount is to be increased. The upper limit of the extraction time may be, but is not limited to, for example, about 120 minutes. During the extraction step, the fungal cells and the extraction solvent that have been brought into contact with each other may be stirred, may be shaken, or may be left to stand as appropriate.

By exposing the fungal cells, and/or the culture liquid containing the fungal cells to a high temperature condition during or before the extraction step as described above, the fungal cells can be killed, and/or enzymes having an action to degrade MAAs in the fungal cells can be inactivated, so that the amount of MAAs recovered can be improved. The high temperature condition to which the culture liquid is exposed may be, for example, not less than 40°C or not less than 50°C.

Therefore, in another aspect of the extraction method of the present disclosure, the survival rate of the fungal cells after the extraction step may be 0% to 20%, 0% to 10%, or 0% to 5% compared to those cells after the culture step. The survival rate is not limited as long as the survival rate after the extraction step is within this range. However, in cases where the survival rate of the fungal cells is such a low rate also before the extraction step or during the extraction step, degradation of MAAs can be expected to be suppressed to improve the amount of MAAs recovered.

After the extraction step, the obtained extract may be subjected to removal of foreign substances, fractionation, purification, and the like by well-known methods.

The MAAs extracted in the present disclosure are not limited as long as they are produced from a black yeast of the family Dothioraceae of the order Dothideales. Usually, the MAAs are ring compounds that absorb ultraviolet. Specific examples of the MAAs may include compounds containing cyclohexanone or cyclohexenimine as a central ring; or compounds in which various functional groups such as amino acids are bound to the central ring.

More specific examples of the MAAs include mycosporine-glutamine, mycosporine-glutaminol, mycosporine-glutamic acid, mycosporine-glutamicol, mycosporine-hydroxyglutamicol, mycosporine-glutaminol-glucoside (MGGnol), mycosporine-glutamicol-glucoside (MGGcol), gadusol, deoxygadusol, mycosporine-glycine, mycosporine-taurine, mycosporine-alanine, mycosporine-serine, mycosporine-serinol, mycosporine-2-glycine, palythinol, palythenic acid, mycosporine-methylamine-threonine, mycosporine-glycine-valine, palythine, asterina-330, shinorine, porphyra-334, euhalothece-362, mycosporine-ornithine, mycosporine-lysine, mycosporine-glutamic acid-glycine, mycosporine-methylamine-serine, mycosporine-taurine, palythene, palythine-serine, palythine-serine-sulfate, and usujirene. The MAAs may be one of, or two or more of these. The term "MAAs" in the present disclosure may mainly mean, among these, MGGnol and/or MGGcol (hereinafter collectively referred to as "MGGs"), which are especially known to be produced by a black yeast of the family Dothioraceae of the order Dothideales, produced by the genus *Aureobasidium,* the genus *Hormonema* (*Sydowia*)*,* or the genus *Hortaea,* or produced by *Aureobasidium pullulans* and contained in its fungal cells (Patent Document 1, Non-Patent Document 2).

These MAAs are usually water-soluble. Further, as shown in the Examples below, it has been confirmed that, even under high temperature conditions, they are not lost due to degradation or the like, and can be obtained in a sufficient amount. Conventionally, it has been expected that the ultraviolet-absorbing ring structures of MAAs may be easily degraded by heat, and hence that MAAs can be hardly obtained in cases where they have undergone heat treatment. However, it was surprisingly discovered that the method of the present disclosure enables maintenance of the ultraviolet-absorbing capacity and recovering of MAAs without a decrease in their amount.

In general, MAAs have their ultraviolet-absorbing capacity due to structures containing a central ring such as cyclohexanone and/or cyclohexenimine. Therefore, confirmation of the presence of MAAs in an extract, and measurement of the amount and concentration of MAAs in an extract can be carried out using a light wavelength within the ultraviolet region. For example, these can be carried out by measuring the ultraviolet absorption (280 to 370 nm) of MAAs in the extract using a spectrophotometer (Patent Document 1, Non-Patent Document 2). In particular, measurement of the amount of MGGnol and MGGcol can be suitably carried out by measurement of the absorbance (ABS) at the maximum wavelength of MGGnol, 310 nm, using a spectrophotometer, wherein the concentration of MGGs in the measurement sample can be calculated according to the following: the molar absorption coefficient of MGGnol: 25,000, ABS 1: MGGnol 18.7 µg/mL. Although the extract of the present disclosure may also contain MAAs other than MGGnol and MGGcol, the amount of MGGnol and MGGcol is thought to account for most of the amount of MAAs measured by the absorbance at a wavelength of 310 nm in the extract of the present disclosure.

The extraction method of the present disclosure can be incorporated into a method of producing a polar solvent extract of cultured fungal cells of a black yeast of the family Dothioraceae of the order Dothideales, the method comprising extraction of MAAs. The polar solvent extract herein may be a water extract.

The polar solvent extract obtained by such a production method may contain a high concentration of MAAs. Specifically, the MAAs may be contained at not less than 0.06 g/L, not less than 0.1 g/L, or not less than 0.51 g/L in terms of the MAA yield per fungal cell culture liquid that is the extraction source.

The extraction method of the present disclosure enables extraction of MAAs from fungal cells with a higher recovery compared to conventional extraction at low temperature that uses water extraction and/or extraction with a polar solvent. As demonstrated in the Examples below, the amount of MAAs recovered can be considered to account for almost the entire MAAs produced by, and contained in, the fungal cells. In addition, the increased amount of MAAs recovered can be secured by the fact that degradation of MAAs by enzymes contained in the living fungal cells can be suppressed through the heat treatment.

The MAA-containing polar solvent extract of the present disclosure may be included in various compositions together with optional components. The route of administration (ingestion) of each composition is not limited, and may be, for example, transdermal administration, oral administration, ocular instillation, or nasal instillation. Examples of the mode of the composition include pharmaceuticals, quasi-drugs, foods and beverages (such as foods with function claims, and supplements), and cosmetics. In particular, taking the advantage of its ultraviolet-absorbing capacity, the composition may be included in sunscreen cosmetics.

The present description also provides a method of producing an MAA-containing composition. The production method is based on the discovery that MAAs have heat resistance that allows maintenance of the ultraviolet-absorbing capacity. The method is characterized in that it comprises a heating step of heating an MAA-containing composition.

The heating step may be carried out in any step in the process of the production of the MAA-containing composition.

Specifically, the production method may also encompass a mode including (a) the extraction step of extracting an MAA fraction into a polar solvent at not less than 40°C; and/or a mode including (b) the heating step of collecting, before the extraction step, fungal cells from the culture liquid after the culture step, and heating the collected fungal cells; in the extraction method of the present disclosure. In that case, the MAA-containing composition to be heated refers to the fungal cells themselves, and/or a culture liquid containing the fungal cells. Further, in that case, the heating conditions in the heating step may be in accordance with the description for (a) the extraction step of extracting an MAA fraction into a polar solvent at not less than 40°C, and the description for (b) heating of the fungal cells. In this mode, the heating step can be a step that improves the efficiency of extraction of MAAs from the fungal cells.

The production method may also encompass a mode in which MAAs obtained by an arbitrary method are included in a composition, followed by subjecting the resulting composition to the heating step. Such a mode can be, for example, a mode in which sterilization is carried out, or in which enzymes in the composition are inactivated, in the production of an MAA-containing composition such as a cosmetic.

In this mode, the MAAs included in the composition may be obtained by any method, and may be MAAs extracted from a black yeast of the family Dothioraceae of the order Dothideales or from another microorganism. Regarding the extraction conditions, the extraction solvent is not limited to a polar solvent, and any extraction solvent may be used. The extraction may be carried out at any temperature for any length of time. Further, before the extraction, the microorganism may or may not be subjected to an optional pretreatment such as heat drying. Further, a production method in this mode may be carried out, for example, for a composition containing an MAA fraction obtained by a method comprising: a culture step of culturing a black yeast of the family Dothioraceae of the order Dothideales; and an extraction step of extracting an MAA fraction from cultured fungal cells of the black yeast.

The heating condition in this mode is usually not limited as long as the sterilization and/or enzyme inactivation can be achieved. For example, the heating may be carried out at 40°C to 95°C, 50°C to 90°C, or 60°C to 85°C. Further, an apparatus such as an autoclave may be used to perform heating to not less than 90°C, or to a temperature of 100°C to 135°C, 110°C to 130°C, or 120°C to 125°C.

The present production method may further comprise a pressurization step of pressurizing the MAA-containing composition. The pressurization step may be carried out before, after, or at the same time as the heating step. The degree of the pressurization may be not less than 0.10 MPa, not less than 0.12 MPa, not less than 0.15 MPa, or not less than 0.20 MPa.

Unless otherwise defined, all technical terms and scientific terms used herein have the same meanings as those generally understood by those skilled in the art to which the present disclosure belongs. Any of the methods and materials similar or equivalent to the methods and materials described herein can be used in the practice or testing of the present disclosure, and representative exemplary methods and materials are described herein.

It is understood that, when a range of values is provided, the individual values between the upper limit and the lower limit of the range (to the tenth of the unit of the lower limit unless the context clearly dictates otherwise), and any other described values or intervening values within the described range are encompassed within the scope of the invention presented in the present disclosure. The upper and lower limits of a narrower range thereof may be independently included in the narrower range. They are also encompassed within the scope of the invention presented in the present disclosure, and subject to any specifically excluded limit in the described range. In cases where a described range includes one or both of the limits, ranges excluding one or both of those included limits are also included in the invention presented in the present disclosure.

This present disclosure is not limited to the specific modes described. Since the scope of the invention presented in the present disclosure is considered to be limited only by the appended claims, it should also be understood that the terminology used herein is merely for the purpose of describing particular modes, and is not intended to be limiting.

As will be apparent to those skilled in the art upon reading this disclosure, each of the individual modes described herein has discrete components and features that may be readily separated from or combined with features of several other modes without departing from the scope and the spirit of the present disclosure. Any cited method may be implemented in the order of the cited events or in any other order that is logically possible and consistent.

All publications and patents cited herein are hereby incorporated by reference as if each individual publication or patent were specifically and individually indicated to be incorporated by reference, and the publications are incorporated herein by reference to disclose and describe the methods and/or materials with which the publications are cited in connection. The citation of any publication is for its disclosure prior to the filing date, and should not be construed as an admission that, by virtue of prior invention, the invention described in the present disclosure is not entitled to antedate such publication. Further, the dates of publication provided may be different from the actual publication dates, and the actual publication dates may need to be individually confirmed.

The following Examples are given for the purpose of exemplifying various modes of the present disclosure, and are not intended to limit the present disclosure in any fashion. Those skilled in the art will readily appreciate that the present disclosure is well adapted to carry out the mentioned objects to achieve the final goal and the advantages mentioned, and also well adapted to carry out the inherent objects of the present description to achieve the final goal and the advantages. The present Examples, along with the methods described herein, are representative modes at present. They are exemplary, and not intended to limit the scope of the present disclosure. Modifications and other uses encompassed within the spirit of the present disclosure as defined by the claims will occur to those skilled in the art.

### EXAMPLES

The invention presented in the present disclosure is described below by way of Examples, but the invention presented in the present disclosure is not limited by these Examples. Unless otherwise specified, the units are on a weight basis.

### <Example 1> Hot Water Extraction from Lyophilized Fungal Cells

### (1) Culture

With reference to Example 1 of Patent Document 1, a liquid medium (hereinafter referred to as "medium A") was prepared by mixing 3% by weight glucose, 0.5% by weight soy peptone (manufactured by Solabia Biokar Diagnostics), 0.1% by weight sodium ascorbate, 0.1% by weight monopotassium phosphate, 0.05% by weight magnesium sulfate heptahydrate, 0.3% by weight yeast extract (manufactured by TASTONE 154AG SENSIENT), and a 0.1% by weight metal salt solution (0.5% by weight ferrous sulfate heptahydrate, 0.05% by weight calcium chloride dihydrate, 0.01% by weight copper sulfate pentahydrate, 0.05% by weight zinc sulfate heptahydrate, and 0.05% by weight manganese chloride tetrahydrate) in water. After placing 100 mL of the liquid medium in a 500-mL Erlenmeyer flask, heat sterilization was carried out at 121°C for 15 minutes.

Further, as a medium containing neither sodium ascorbate nor peptone, a liquid medium (hereinafter referred to as "medium B") was prepared by mixing 3% by weight glucose and 1% by weight yeast extract (manufactured by TASTONE 154AG SENSIENT) in water. After placing 100 mL of the liquid medium in a 500-mL Erlenmeyer flask, heat sterilization was carried out at 121°C for 15 minutes.

*Aureobasidium pullulans* (the 6353 strain, the 100716 strain, the 106987 strain, the 4464 strain, the 7757 strain, 108784, and the 114573 strain) obtained from NBRC was inoculated to PDA plates (potato dextrose agar medium, granular (manufactured by Nissui Pharmaceutical Co., Ltd.)), and cultured at 25°C for 4 days. A loopful of grown fungal cells were inoculated to the sterilized liquid medium A or B, and shake culture was performed at 25°C at 100 rpm for 7 days.

The entire culture liquid after the culture was centrifuged at 8000 rpm for 5 minutes to collect the fungal cells separated from the supernatant.

The fungal cells were lyophilized. The dry fungal cell weight was measured to calculate the amount of fungal cells per 1 L of the culture liquid.

### (2) Extraction

After taking 20 mg ± 2 mg of the collected fungal cells into a lidded test tube, 3 mL of an extraction solvent was added thereto, and the fungal cells were suspended with stirring. As the extraction solvent, water at 25°C, 40°C, 50°C, 60°C, 80°C, or 121°C, or 10% aqueous ethanol solution or 20% aqueous ethanol solution at 25°C was used. The test tubes to which each extraction solvent at 25°C (normal temperature) was added were shaken using a shaker throughout the extraction period. The test tubes to which water at 40°C, 50°C, 60°C, or 80°C was added were heated at the predetermined temperature using a block heater with stirring by vortexing at 15-minute intervals throughout the extraction period. For the condition where water at 121°C was used, a test tube to which water was added was placed in an autoclave, and heated at 121°C for 15 minutes. The extraction time was set to 15 minutes, 30 minutes, 60 minutes, or 120 minutes.

After the predetermined extraction time has passed, each test tube was left to stand to allow the fungal cells to precipitate, and 1 mL of the supernatant was dispensed into a centrifuge tube, followed by performing centrifugation at 12,000 rpm for 3 minutes, and collecting the resulting supernatant to provide a measurement sample.

### (3) Measurement of Extracted Amount

It is known that the cultured fungal cells produce and contain MGGnol and/or MGGcol therein, and that the amount of MGGnol can be measured at a wavelength within the ultraviolet region since it has ultraviolet-absorbing capacity (Patent Document 1, Non-Patent Document 2).

For each measurement sample obtained in (2), ABS at the maximum absorption wavelength of MGGnol, 310 nm, was measured using a spectrophotometer. The concentration of MGGs in the measurement sample was calculated according to the following: the molar absorption coefficient of MGGnol: 25,000, ABS 1: MGGnol 18.7 µg/mL; and then the MGG content per unit amount of fungal cells was calculated. Since MGGcol also absorbs light at a wavelength of 310 nm, MGGcol may also be included in the amount of MGGs in the following Examples.

### (4) Measurement of Survival Rate

The fungal suspension after the extraction in (2) was diluted as appropriate, and inoculated to a PDA plate, followed by performing culture at 25°C. The survival rate was calculated by taking, as 100%, the number of grown colonies in the suspension at the start of the extraction (extraction time, 0 minutes) in the case where the extraction was carried out with water at 25°C.

### (5) Results

Table 1 shows, for each fungal strain, the MGG content (mg/g) (the amount of MGGs per 1 g of fungal cells) and the MGG yield (g/L) (the amount of MGGs per 1 L of the culture liquid, MGG content × dried fungal cells) in the case where the extraction was carried out with water at 80°C for 30 minutes. The fungal cells cultured in medium B showed higher MGG yields than those cultured in medium A. Further, in the NBRC6353 strain, the MGG content was much higher in the case where the culture was performed with medium B than in the case where the culture was performed with medium A. Further, the color of the fungal cells of the NBRC6353 strain was black in the case where they were cultured in medium A, but was whitish gray in the case where they were cultured in medium B. The black color of the fungal cells of the NBRC6353 strain is thought to be due to melanin. The colors of the fungal cells of the NBRC106987 strain and the NBRC10716 strain cultured in medium B were pale orange.

Tables 2 and 3 show, for each fungal strain, the MGG content and the extraction rate achieved under each extraction condition, wherein the extraction rate was calculated taking, as 100%, the MGG content in the case where the extraction was carried out with water at 25°C for 120 minutes (Comparative Example). For the fungal cells cultured in either medium A or medium B, the extraction rate was found to be higher in the cases where the extraction solvent was heated to not less than 40°C than in the Comparative Example. In the case where the extraction was carried out with 10% ethanol at 25°C for 120 minutes, the extraction rate was higher or lower than in the Comparative Example depending on the fungal strain. In the cases where the extraction was carried out with water at 80°C for 15 minutes, and the cases where the extraction was carried out with water at 121°C for 15 minutes (that is, the sterilization conditions), the peak waveform at 310 nm in the measurement sample was not different from those in the cases of extraction at lower temperatures, indicating that heating does not cause a decrease in MGGs. Under the conditions at 25°C, the aqueous ethanol solutions showed higher extraction rates than water. On the other hand, in the cases where the extraction was carried out with water at not less than 50°C, a higher extraction rate could be achieved in a shorter time than in the cases where an aqueous ethanol solution at 25°C was used.

The extraction rate of MGGs from the fungal cells of the NBRC6353 strain was not less than two times higher in the cases where the extraction was carried out with water at 80°C than in the case where the extraction was carried out with water at 25°C for 120 minutes. Fungal cells of yeasts of the genus *Aureobasidium* are known to have a black color when they are cultured for a long time. Further, according to the above results, extraction of MGGs from melanin-containing fungal cells is considered to be more difficult. However, it can be seen that the method of the present disclosure enables efficient extraction of MGGs even from black fungal cells that have appeared as a result of culturing for a long time.

Table 4 shows, for each fungal strain, the survival rate under each extraction condition. In the case where the extraction was carried out with water at 25°C for 120 hours, most fungal cells survived until the completion of the extraction. It was thus suggested that, under this condition, the fungi survive during and also after the extraction step, resulting in a decrease in the MGG yield due to utilization of MGGs.

**[Table 1]**

| Table 1: Example 1 | | | | | | |
|---|---|---|---|---|---|---|
| Aureobasidium pullulans | Medium A | | | Medium B | | |
| | Dried fungal cells | MGGs content | MGGs yield | Dried fungal cells | MGGs content | MGGs yield |
| | (g/L) | (mg/g) | (g/L) | (g/L) | (mg/g) | (g/L) |
| NBRC 4464 | 8.1 | 8.5 | 0.07 | - | - | - |
| NBRC 6353 | 9.4 | 4.6 | 0.04 | 13.0 | 13.5 | 0.18 |
| NBRC 7757 | 7.9 | 7.9 | 0.06 | - | - | - |
| NBRC 100716 | 8.2 | 13.1 | 0.11 | 10.1 | 17.4 | 0.18 |
| NBRC 106987 | 8.1 | 15.3 | 0.12 | 9.8 | 14.6 | 0.14 |
| NBRC 114573 | 8.5 | 2.9 | 0.03 | - | - | - |
| NBRC 108784 | 9.7 | 6.4 | 0.06 | - | - | - |

**[Table 2]**

| Table 2: Example 1 (Medium A) | | | | | | | | | | |
|---|---|---|---|---|---|---|---|---|---|---|
| Aureobasidium pullulans | Solvent | Time | 15 minutes | | 30 minutes | | 60 minutes | | 120 minutes | |
| | | Temperature | MGGs (mg/g) | Extraction rate | MGGs (mg/g) | Extraction rate | MGGs (mg/g) | Extraction rate | MGGs (mg/g) | Extraction rate |
| | Water | 25°C | | | | | | | 11.1 | 100% |
| | | 40°C | | | | | 12.0 | 108% | 12.6 | 114% |
| | | 50°C | 12.5 | 113% | 13.6 | 123% | 14.3 | 129% | 14.9 | 134% |
| NBRC 100716 | | 60°C | 14.6 | 132% | 14.9 | 134% | 15.4 | 139% | 15.3 | 138% |
| | | 70°C | 16.2 | 146% | 16.2 | 146% | 16.1 | 145% | | |
| | | 80°C | 16.1 | 145% | 16.3 | 147% | 16.7 | 150% | | |
| | | 121°C | 15.7 | 141% | | | | | | |
| | 10% Ethanol | 25°C | | | | | | | 10.9 | 98% |
| | 20% Ethanol | 25°C | | | | | | | 11.6 | 105% |
| | Water | 25°C | | | | | | | 10.6 | 100% |
| | | 40°C | | | | | 12.8 | 121% | 15.8 | 149% |
| | | 50°C | 12.5 | 118% | 14.2 | 134% | 15.2 | 143% | 16.2 | 153% |
| NBRC 106987 | | 60°C | 15.1 | 142% | 15.2 | 143% | 15.9 | 150% | 16.2 | 153% |
| | | 70°C | 15.5 | 146% | 15.8 | 149% | 16.2 | 153% | | |
| | | 80°C | 15.9 | 150% | 15.9 | 150% | 16.3 | 154% | | |
| | | 121°C | 16.6 | 157% | | | | | | |
| | 10% Ethanol | 25°C | | | | | | | 12.8 | 121% |
| | 20% Ethanol | 25°C | | | | | | | 14.7 | 139% |
| NBRC 6353 | Water | 25°C | | | | | | | 4.2 | 100% |
| | | 40°C | | | | | 4.8 | 114% | 5.7 | 136% |
| | | 50°C | | | 5.7 | 136% | 5.4 | 129% | | |
| | | 60°C | | | 5.8 | 138% | 6.1 | 145% | | |
| | | 80°C | 7.3 | 174% | 7.4 | 176% | | | | |
| | | 121°C | 8.0 | 190% | | | | | | |
| | 10% Ethanol | 25°C | | | | | | | 5.3 | 126% |
| | 20% Ethanol | 25°C | | | | | | | 6.0 | 143% |
| NBRC 4464 | Water | 25°C | | | | | | | 8.0 | 100% |
| | | 80°C | 10.5 | 131% | 11.2 | 140% | | | | |
| | | 121°C | 10.9 | 136% | | | | | | |
| | 10% Ethanol | 25°C | | | | | | | 7.5 | 94% |
| | 20% Ethanol | 25°C | | | | | | | 8.1 | 101% |
| NBRC 7757 | Water | 25°C | | | | | | | 8.1 | 100% |
| | | 80°C | 10.1 | 125% | 10.8 | 133% | | | | |
| | | 121°C | 11.2 | 138% | | | | | | |
| | 10% Ethanol | 25°C | | | | | | | 8.9 | 110% |
| | 20% Ethanol | 25°C | | | | | | | 10.5 | 130% |
| NBRC 108784 | Water | 25°C | | | | | | | 4.7 | 100% |
| | | 80°C | 7.2 | 153% | 7.5 | 160% | | | | |
| | | 121°C | 7.1 | 151% | | | | | | |
| | 10% Ethanol | 25°C | | | | | | | 6.2 | 132% |
| | 20% Ethanol | 25°C | | | | | | | 7.1 | 151% |

**[Table 3]**

| Table 3: Example 1 (Medium B) | | | | | | | | | | |
|---|---|---|---|---|---|---|---|---|---|---|
| Aureobasidium pullulans | Solvent | Time | 15 minutes | | 30 minutes | | 60 minutes | | 120 minutes | |
| | | Temperature | MGGs (mg/g) | Extraction rate | MGGs (mg/g) | Extraction rate | MGGs (ma/a) | Extraction rate | MGGs (mg/g) | Extraction rate |
| NBRC 100716 | Water | 25°C | | | | | | | 11.2 | 100% |
| | | 40°C | | | | | 16.2 | 145% | 17.3 | 154% |
| | | 50°C | | | 16.3 | 146% | 17.1 | 153% | 17.1 | 153% |
| | | 80°C | 17.2 | 154% | 17.0 | 152% | 17.4 | 155% | | |
| NBRC 106987 | Water | 25°C | | | | | | | 11.7 | 100% |
| | | 40°C | | | | | 14.2 | 121% | 14.3 | 122% |
| | | 50°C | | | 14.5 | 124% | 14.3 | 122% | 14.4 | 123% |
| | | 80°C | 14.1 | 121% | 14.2 | 121% | 14.6 | 125% | | |
| NBRC 6353 | Water | 25°C | | | | | | | 4.7 | 100% |
| | | 40°C | | | | | 6.4 | 136% | 7.0 | 149% |
| | | 50°C | | | 7.0 | 149% | 7.9 | 168% | 9.8 | 209 |
| | | 80°C | 13.4 | 285% | 13.3 | 283% | 13.2 | 281% | | |

**[Table 4]**

| Aureobasidium pullulans | Solvent | Time | 15 minutes | | 30 minutes | | 60 minutes | | 120 minutes | |
|---|---|---|---|---|---|---|---|---|---|---|
| | | Temperature | Number of living cells | Survival rate | Number of living cells | Survival rate | Number of living cells | Survival rate | Number of living cells | Survival rate |
| NBRC 6353 | Water | 25°C | | | | | | | 12,300,000 | 95% |
| | | 40°C | | | | | | | 180,000 | 1% |
| | | 50°C | | | 5,000 | 0.03846% | 5,000 | 0.0385% | | |
| | | 60°C | | | 1,380 | 0.01062% | 40 | 0.0003% | | |
| | | 70°C | 2,300 | 0.01769% | 150 | 0.00115% | | | | |
| | | 80°C | 20 | 0.00015% | 50 | 0.00038% | | | | |
| | 10% Ethanol | 25°C | | | | | | | | Mostly grown |
| | 20% Ethanol | 25°C | | | | | | | 0 | 0% |
| NBRC 100716 | Water | 25°C | | | | | | | 16,000,000 | 107% |
| | | 40°C | | | | | | | 300,000 | 2% |
| | | 50°C | | | 3,180 | 0.02120% | 1,010 | 0.0067% | | |
| | | 60°C | | | 1,800 | 0.01200% | 1,530 | 0.0102% | | |
| | | 70°C | 300 | 0.00200% | 60 | 0.00040% | | | | |
| | | 80°C | 18 | 0.00012% | 0 | 0.00000% | | | | |
| | 10% Ethanol | 25°C | | | | | | | | Mostly grown |
| | 20% Ethanol | 25°C | | | | | | | 0 | 0% |
| NBRC 106987 | Water | 25°C | | | | | | | 19,800,000 | 99% |
| | | 40°C | | | | | 5,000 | 0.0250% | 9,000 | 0% |
| | | 50°C | | | 5,000 | 0.02500% | 1,130 | 0.0084% | | |
| | | 60°C | | | 3,000 | 0.02222% | 450 | 0.0017% | | |
| | | 70°C | 3,010 | 0.01505% | 890 | 0.00445% | | | | |
| | | 80°C | 620 | 0.00459% | 350 | 0.00259% | | | | |
| | 10% Ethanol | 25°C | | | | | | | | Mostly grown |
| | 20% Ethanol | 25°C | | | | | | | 0 | 0% |

### <Example 2> Hot Water Extraction from Heat-Dried Fungal Cells 1

### (1) Method

The lyophilized fungal cells obtained by the culture in medium A in (1) of Example 1 were dried at 105°C for 2 hours. According to the weights measured before and after the drying, the water content was found to have hardly decreased (by not more than 1%). After taking 20 mg ± 2 mg of the dried fungal cells into a lidded test tube, extraction was carried out in the same manner as in (2) of Example 1, and then the extraction rate was calculated in the same manner as in (3).

### (2) Results

Table 5 shows, for each fungal strain, the MGG content and the extraction rate achieved under each extraction condition, wherein the extraction rate was calculated taking, as 100%, the content in the case where the extraction was carried out with water at 25°C for 120 minutes (Example 1).

The extraction rate was improved in the cases where the heat drying treatment was carried out before the extraction, compared to the case where lyophilized fungal cells without heat drying treatment were subjected to extraction with water at 25°C for 120 minutes. In the cases where the extraction was carried out with water at 40°C, 60°C, or 80°C, the extraction rate was almost the same as those in the cases where lyophilized fungal cells without heat drying treatment were subjected to extraction using the same extraction solvent (Table 2). It could thus be confirmed that degradation or denaturation of MGGs does not occur even by heating at 105°C for 2 hours.

**[Table 5]**

| Table 5: Example 2 (Medium A) | | | | | | | |
|---|---|---|---|---|---|---|---|
| Aureobasidium pullulans | Drying | Solvent | Time | 60 minutes | | 120 minutes | |
| | | | Temperature | MGGs (mg/g) | Extraction rate | MGGs (mg/g) | Extraction rate |
| NBRC 6353 | Lyophilization (Example 1) | Water | 25°C | | | 4.2 | 100% |
| | 105°C, 2 hours | Water | 25°C | | | 5.1 | 121% |
| | | | 40°C | | | 6.2 | 148% |
| | | | 60°C | 7.3 | 174% | | |
| | | | 80°C | 7.0 | 167% | | |
| | | 20% Ethanol | 25°C | | | 6.7 | 160% |
| NBRC 100716 | Lyophilization (Example 1) | Water | 25°C | | | 11.8 | 100% |
| | 105°C, 2 hours | Water | 25°C | | | 13.2 | 119% |
| | | | 40°C | | | 14.3 | 121% |
| | | | 60°C | 13.6 | 123% | | |
| | | | 80°C | 14.0 | 127% | | |
| | | 20% Ethanol | 25°C | | | 12.8 | 108% |
| NBRC 106987 | Lyophilization (Example 1) | Water | 25°C | | | 10.6 | 100% |
| | 105°C, 2 hours | Water | 25°C | | | 14.6 | 138% |
| | | | 40°C | | | 15.4 | 145% |
| | | | 60°C | 15.6 | 147% | | |
| | | | 80°C | 15.8 | 149% | | |
| | | 20% Ethanol | 25°C | | | 15.4 | 145% |

### <Example 3> Hot Water Extraction from Heat-Dried Fungal Cells 2

### (1) Method

In the same manner as in (1) of Example 1, *Aureobasidium pullulans* (the 100716 strain, the 106987 strain, and the 7757 strain) was subjected to shake culture in medium A at 25°C at 100 rpm for 6 days. The entire culture liquid after the culture was centrifuged at 8000 rpm for 5 minutes, and the precipitated wet fungal cells were collected. After adding 5 mL of water to the collected fungal cells, the fungal cells were suspended, and the resulting suspension was placed in a beaker, followed by drying at 105°C for 2 hours. After taking 20 mg ± 2 mg of the dried fungal cells into a lidded test tube, extraction was carried out in the same manner as in (2) of Example 1, and then the extraction rate was calculated in the same manner as in (3).

### (2) Results

Table 6 shows, for each fungal strain, the MGG content and the extraction rate achieved under each extraction condition, wherein the extraction rate was calculated taking, as 100%, the content in the case where the extraction was carried out with water at 25°C for 120 minutes (Example 1).

Since the wet fungal cells after the culture were dried at 105°C for 2 hours, even the extraction with water at 25°C could achieve almost the same extraction rate as that obtained in the case where the extraction was carried out with water at 80°C for 30 minutes from lyophilized fungal cells without heat drying treatment (Table 2). It could thus be confirmed that degradation or denaturation of MGGs does not occur even by heating of the wet fungal cells at 105°C for 2 hours after the culture.

**[Table 6]**

| Table 6: Example 3 (Medium A) | | | | | | |
|---|---|---|---|---|---|---|
| Aureobasidium pullulans | Drying | Solvent | Temperature | Time | MGGs (mg/g) | Extraction rate |
| NBRC 7757 | Lyophilization (Example 1) | Water | 25°C | 120 minutes | 8.1 | 100% |
| | Heat drying | Water | 25°C | 120 minutes | 10.8 | 134% |
| | | | 40°C | 120 minutes | 12.5 | 155% |
| | | | 50°C | 30 minutes | 13.1 | 162% |
| | | | 60°C | 30 minutes | 12.1 | 151% |
| | | | 80°C | 30 minutes | 12.4 | 154% |
| | | 20% Ethanol | 25°C | 120 minutes | 10.4 | 128% |
| NBRC 100716 | Lyophilization (Example 1) | Water | 25°C | 120 minutes | 11.1 | 100% |
| | Heat drying | Water | 25°C | 120 minutes | 16.7 | 151% |
| | | | 40°C | 120 minutes | 16.7 | 151% |
| | | | 50°C | 30 minutes | 17.5 | 158% |
| | | | 60°C | 30 minutes | 16.9 | 153% |
| | | | 80°C | 30 minutes | 17.0 | 154% |
| | | 20% Ethanol | 25°C | 120 minutes | 16.3 | 147% |
| NBRC 106987 | Lyophilization (Example 1) | Water | 25°C | 120 minutes | 10.6 | 100% |
| | Heat drying | Water | 25°C | 120 minutes | 18.7 | 176% |
| | | | 40°C | 120 minutes | 18.8 | 177% |
| | | | 50°C | 30 minutes | 19.7 | 186% |
| | | | 60°C | 30 minutes | 19.4 | 183% |
| | | | 80°C | 30 minutes | 18.4 | 173% |
| | | 20% Ethanol | 25°C | 120 minutes | 18.8 | 177% |

### <Example 4> Extraction from Fungal Cells in Culture Liquid by Heating of Culture Liquid

### (1) Method

In the same manner as in Example 3, *Aureobasidium pullulans* (the 100716 strain, the 106987 strain, and the 7757 strain) was cultured in medium A, to obtain culture liquids. Further, in the same manner as in (1) of Example 1, *Aureobasidium pullulans* (the 100716 strain, the 106987 strain, and the 6353 strain) was cultured in medium B, to obtain culture liquids. Each culture liquid was placed in a beaker, and stirred using a stirrer in order to uniformly suspend the cells. The culture liquid was aliquoted in 3-mL volumes into 15-mL centrifuge tubes using a measuring pipette, and the centrifuge tubes were placed on a block heater at 40°C, 50°C, or 60°C, followed by leaving the tubes to stand for 30 minutes, 60 minutes, or 120 minutes to carry out extraction treatment. In order to avoid precipitation of the fungal cells, the culture liquid was stirred by vortexing at 15-minute intervals. Thereafter, the supernatant of the culture liquid was diluted 20-fold, and then the MGG concentration was measured in the same manner as in (3) of Example 1.

### (2) Results

Tables 7 and 8 show, for each fungal strain, the MGG concentration in the dilution of the culture liquid obtained after the treatment under each extraction condition.

In view of the amounts of MGGs extracted from the dried fungal cells in the above Examples, it could be confirmed that almost the entire MGGs produced by the fungal cells were extracted in the case where the culture liquid was heated at 50°C for 60 minutes.

In the NBRC100716 strain, the amount of MGGs in the case of extraction by heating of the culture liquid at 40°C for 120 minutes was only about 30% compared to that in the case of extraction by heating at 60°C for 60 minutes. Even in the case where the extraction was carried out at 50°C for 60 minutes, the extracted amount was small. On the other hand, in the NBRC7757 strain and the NBRC106987 strain, the amount of MGGs extracted by heating at 40°C for 120 minutes was about 70 to 80% compared to that extracted by heating at 60°C for 60 minutes.

**[Table 7]**

| Table 7: Example 4 (Medium A) | | | | |
|---|---|---|---|---|
| Aureobasidium pullulans | Temperature | Time | ABS (310nm) | MGGs concentration (µg/mL) |
| NBRC 7757 | 40°C | 120 minutes | 0.306 | 114 |
| | 50°C | 30 minutes | 0.366 | 137 |
| | 50°C | 60 minutes | 0.365 | 137 |
| | 60°C | 30 minutes | 0.339 | 127 |
| | 60°C | 60 minutes | 0.364 | 136 |
| NBRC 100716 | 40°C | 120 minutes | 0.177 | 66 |
| | 50°C | 30 minutes | 0.201 | 75 |
| | 50°C | 60 minutes | 0.49 | 183 |
| | 60°C | 30 minutes | 0.552 | 206 |
| | 60°C | 60 minutes | 0.558 | 209 |
| NBRC 106987 | 40°C | 120 minutes | 0.431 | 161 |
| | 50°C | 30 minutes | 0.576 | 215 |
| | 50°C | 60 minutes | 0.564 | 211 |
| | 60°C | 30 minutes | 0.598 | 224 |
| | 60°C | 60 minutes | 0.591 | 221 |

**[Table 8]**

| Table 8: Example 4 (Medium B) | | | | |
|---|---|---|---|---|
| Aureobasidium pullulans | Temperature | Time | ABS (310nm) | MGGs concentration (µg/mL) |
| NBRC 6353 | 40°C | 120 minutes | 0.179 | 67 |
| | 50°C | 30 minutes | 0.168 | 63 |
| | 50°C | 60 minutes | 0.602 | 225 |
| | 60°C | 30 minutes | 0.595 | 223 |
| | 60°C | 60 minutes | 0.577 | 216 |
| NBRC 100716 | 40°C | 120 minutes | 0.216 | 81 |
| | 50°C | 30 minutes | 0.184 | 69 |
| | 50°C | 60 minutes | 0.313 | 117 |
| | 60°C | 30 minutes | 0.652 | 244 |
| | 60°C | 60 minutes | 0.676 | 253 |
| NBRC 106987 | 40°C | 120 minutes | 0.213 | 80 |
| | 50°C | 30 minutes | 0.160 | 60 |
| | 50°C | 60 minutes | 0.365 | 137 |
| | 60°C | 30 minutes | 0.388 | 145 |
| | 60°C | 60 minutes | 0.498 | 186 |

### <Example 5> Hot Water Extraction from Lyophilized Fungal Cells

*Sydowia polyspora* (the 107026 strain, the 107027 strain, and the 107028 strain) obtained from NBRC was inoculated to medium B prepared in the same manner as in Example 1, and shake culture was carried out at 25°C at 100 rpm for 7 days. In the same manner as in Example 1, the cultured fungal cells were collected and lyophilized, followed by calculation of the amount of fungal cells per 1 L of the culture liquid. It should be noted that the anamorph of *Sydowia polyspora* corresponds to *Hormonema macrosporum.*

The extraction operation was carried out in the same manner as in Example 1 except that the amount of fungal cells taken into the lidded test tube was 50 mg ± 5 mg, and thereafter, the extracted amount was measured. Further, the survival rate was measured in the same manner as in Example 1.

Table 9 shows, for each fungal strain, the MGG content (mg/g) (the amount of MGGs per 1 g of fungal cells) and the MGG yield (g/L) (the amount of MGGs per 1 L of the culture liquid, MGG content × dried fungal cells) in the case where the extraction was carried out with water at 80°C for 30 minutes.

Table 10 shows, for each fungal strain, the MGG content and the extraction rate achieved under each extraction condition, wherein the extraction rate was calculated taking, as 100%, the MGG content in the case where the extraction was carried out with water at 25°C for 120 minutes (Comparative Example). Similarly to the cases of the yeasts of the genus *Aureobasidium,* extraction with water at not less than 40°C improved the extraction rate.

Table 11 shows, for each fungal strain, the survival rate under each extraction condition. Similarly to the cases of the yeasts of the genus *Aureobasidium,* the extraction rate increased when the extraction was carried out under conditions where the survival rate was not more than 20%.

**[Table 9]**

| Table 9: Example 5 (Medium B) | | | |
|---|---|---|---|
| Sydowia polyspora | Dried fungal cells | MGGs content | MGGs yield |
| | (g/L) | (mg/g) | (g/L) |
| NBRC 107026 | 12.4 | 5.0 | 0.06 |
| NBRC 107027 | 13.8 | 5.3 | 0.07 |
| NBRC 107028 | 14.6 | 6.2 | 0.09 |

**[Table 10]**

| Table 10: Example 5 (Medium B) | | | | | | | | | | |
|---|---|---|---|---|---|---|---|---|---|---|
| Sydowia polyspora | Solvent | Time | 15 minutes | | 30 minutes | | 60 minutes | | 120 minutes | |
| | | Temperature | MGGs (mg/g) | Extraction rate | MGGs (mg/g) | Extraction rate | MGGs (mg/g) | Extraction rate | MGGs (mg/g) | Extraction rate |
| NBRC 107026 | Water | 25°C | | | | | | | 2.4 | 100% |
| | | 40°C | | | | | | | 3.7 | 154% |
| | | 50°C | | | | | 4.6 | 192% | 4.8 | 200% |
| | | 60°C | | | 4.3 | 179% | 4.6 | 192% | | |
| | | 80°C | | | 4.9 | 204% | 4.9 | 204% | | |
| | | 121°C | 4.9 | 204% | | | | | | |
| | 20% Ethanol | 25°C | | | | | | | 4.2 | 175% |
| NBRC 107027 | Water | 25°C | | | | | | | 3.1 | 100% |
| | | 40°C | | | | | | | 3.9 | 126% |
| | | 50°C | | | | | 4.9 | 158% | 5.3 | 171% |
| | | 60°C | | | 5.3 | 171% | 5.4 | 174% | | |
| | | 80°C | | | 5.2 | 168% | 5.3 | 171% | | |
| | | 121°C | 5.3 | 171% | | | | | | |
| | 20% Ethanol | 25°C | | | | | | | 5.3 | 171% |
| NBRC 107028 | Water | 25°C | | | | | | | 4.4 | 100% |
| | | 40°C | | | | | 5.8 | 132% | 4.7 | 107% |
| | | 50°C | | | 5.8 | 132% | 5.9 | 134% | 6.1 | 139% |
| | | 60°C | | | 6.1 | 139% | 6.1 | 139% | | |
| | | 121°C | 6.0 | 136% | | | | | | |
| | 20% Ethanol | 25°C | | | | | | | 5.7 | 130% |

**[Table 11]**

| Table 11: Example 5 (Medium B) | | | | | | | | | |
|---|---|---|---|---|---|---|---|---|---|
| Sydowia polyspora | Temperature | 60 minutes | | | | 120 minutes | | | |
| | | Dilution | Colony number | Number of living | Survival rate | Dilution | Colony number | Number of living cells | Survival rate |
| NBRC 107026 | 25°C | | | | | 10000 | 315 | 31,500,000 | 100.0% |
| | 40°C | | | | | 3000 | 6 | 180,000 | 1.4% |
| | 50°C | | | | | 100 | 6 | 6,000 | 0.0% |
| | 60°C | 1 | 420 | 4,200 | 0.013% | | | | |
| NBRC 107027 | 25°C | | | | | 10000 | 602 | 60,200,000 | 100.0% |
| | 40°C | | | | | 3000 | 7 | 210,000 | 1.6% |
| | 50°C | | | | | 100 | 21 | 21,000 | 0.2% |
| | 60°C | 1 | 327 | 3,270 | 0.005% | | | | |
| NBRC 107028 | 25°C | | | | | 10000 | 245 | 24,500,000 | 100.0% |
| | 40°C | | | | | 3000 | 6 | 180,000 | 1.4% |
| | 50°C | | | | | 100 | 0 | 0 | 0.0% |
| | 60°C | 1 | 138 | 1,380 | 0.006% | | | | |

### <Example 6> Hot Water Extraction from Heat-Dried Fungal Cells

In the same manner as in Example 5, *Sydowia polyspora* (the 107026 strain, the 107027 strain, and the 107028 strain) was cultured in medium B, and the fungal cells were collected and lyophilized. The lyophilized fungal cells were heat-dried in the same manner as in Example 2, and the extraction operation was carried out in the same manner as in Example 5, followed by measurement of the extracted amount.

Table 12 shows, for each fungal strain, the MGG content and the extraction rate achieved under each extraction condition, wherein the extraction rate was calculated taking, as 100%, the content in the case where the extraction was carried out with water at 25°C for 120 minutes (Example 5).

Also in the yeasts of the genus *Hormonema (Sydowia),* the extraction rate was improved due to death of the yeasts or breakage of the fungal cells in the cases where the heat drying treatment was carried out before the extraction, compared to the case where lyophilized fungal cells without heat drying treatment were subjected to extraction with water at 25°C for 120 minutes. The extraction rate was almost the same between the cases where hot water extraction was carried out for lyophilized fungal cells that had been subjected to heat drying treatment, and the cases where extraction was carried out with water at 80°C for lyophilized fungal cells without heat drying treatment (Table 10). It could thus be confirmed that degradation or denaturation of MGGs does not occur even by heating at 105°C for 2 hours.

**[Table 12]**

| Table 12: Example 6 (Medium B) | | | | | | |
|---|---|---|---|---|---|---|
| Sydowia polyspora | Drying | Time | 60 minutes | | 120 minutes | |
| | | Temperature | MGGs (mg/g) | Extraction rate | MGGs (mg/g) | Extraction rate |
| NBRC 107026 | Lyophilization (Example 5) | 80°C | | | 4.9 | 100% |
| | 105°C, 2 hours | 25°C | | | 4.3 | 88% |
| | | 40°C | | | 4.5 | 92% |
| | | 50°C | 4.8 | 98% | | |
| NBRC 107027 | Lyophilization (Example 5) | 80°C | | | 5.2 | 100% |
| | 105°C, 2 hours | 25°C | | | 4.6 | 88% |
| | | 40°C | | | 4.9 | 94% |
| | | 50°C | 5.2 | 100% | | |
| NBRC 107028 | Lyophilization (Example 5) | 80°C | | | 6.1 | 100% |
| | 105°C, 2 hours | 25°C | | | 5.4 | 89% |
| | | 40°C | | | 5.8 | 95% |
| | | 50°C | 6.0 | 98% | | |

### <Example 7> Extraction from Fungal Cells in Culture Liquid by Heating of Culture Liquid

In the same manner as in Example 5, *Sydowia polyspora* (the 107026 strain, the 107027 strain, and the 107028 strain) was cultured in medium B, to obtain culture liquids. In the same manner as in Example 4, extraction treatment from each culture liquid was carried out. The supernatant of the culture liquid after the extraction operation was diluted 10-fold, and then the extracted amount was measured in the same manner as in Example 1.

Table 13 shows, for each fungal strain, the MGG concentration in the dilution of the culture liquid obtained after the treatment under each extraction condition.

It could be confirmed, also for the yeasts of the genus *Hormonema (Sydowia),* that almost the entire MGGs produced by the fungal cells were extracted by the heating of the culture liquid.

**[Table 13]**

| Table 13: Example 7 (Medium B) | | | | |
|---|---|---|---|---|
| Sydowia polyspora | Temperature | Time | ABS (310nm) | MGGs concentration (µg/mL) |
| NBRC 107026 | 40°C | 120 minutes | 0.357 | 67 |
| | 50°C | 30 minutes | 0.258 | 48 |
| | 50°C | 60 minutes | 0.502 | 94 |
| | 60°C | 30 minutes | 0.412 | 77 |
| | 60°C | 60 minutes | 0.513 | 96 |
| NBRC 107027 | 40°C | 120 minutes | 0.246 | 46 |
| | 50°C | 30 minutes | 0.215 | 40 |
| | 50°C | 60 minutes | 0.429 | 80 |
| | 60°C | 30 minutes | 0.544 | 102 |
| | 60°C | 60 minutes | 0.534 | 100 |
| NBRC 107028 | 40°C | 120 minutes | 0.228 | 43 |
| | 50°C | 30 minutes | 0.386 | 72 |
| | 50°C | 60 minutes | 0.588 | 110 |
| | 60°C | 30 minutes | 0.513 | 96 |
| | 60°C | 60 minutes | 0.540 | 101 |

## Claims

1. A polar solvent extract of cultured fungal cells of a black yeast of the family Dothioraceae of the order Dothideales, the extract comprising a mycosporine-like amino acid (MAA), wherein the MAA yield per fungal cell culture liquid is not less than 0.06 g/L.

2. The polar solvent extract according to claim 1, wherein the MAA comprises mycosporine-glutaminol-glucoside (MGGnol) and/or mycosporine-glutamicol-glucoside (MGGcol).

3. The extract according to claim 1, wherein the polar solvent is water.

4. The extract according to any one of claims 1 to 3, wherein the black yeast is a black yeast belonging to the genus *Aureobasidium,* the genus *Hormonema* (*Sydowia*)*,* or the genus *Hortaea.*

5. A method of extracting an MAA into a polar solvent, the method comprising:
a culture step of culturing a black yeast of the family Dothioraceae of the order Dothideales; and
an extraction step of extracting an MAA fraction from cultured fungal cells of the black yeast;
the method satisfying one or more selected from the following (a), (b), and (c):
(a) in the extraction step, the MAA fraction is extracted into a polar solvent at not less than 40°C;
(b) before the extraction step, a heating step of collecting fungal cells from the culture liquid after the culture step, and heating the collected fungal cells, is carried out; and
(c) in the culture step, the culture is carried out in a medium that does not substantially contain ascorbic acid or a salt thereof, and/or peptone.

6. The method according to claim 5, wherein the (a) is carried out by heating the culture liquid after the culture step to not less than 40°C.

7. The method according to claim 5, wherein the (b) heating step is carried out by heating under one or more conditions selected from the group consisting of (i) to (iii):
(i) a temperature of not less than 105°C;
(ii) a heating time of not less than 2 hours; and
(iii) a pressure of not more than 0.10 MPa.

8. The method according to claim 5, wherein the MAA comprises MGGnol and/or MGGcol.

9. The method according to claim 5, wherein the polar solvent is water.

10. The method according to claim 5, wherein ethanol and/or butyl alcohol is/are not substantially used as the extraction solvent.

11. The method according to any one of claims 5 to 10, wherein the black yeast is a black yeast belonging to the genus *Aureobasidium,* the genus *Hormonema* (*Sydowia*), or the genus *Hortaea.*

12. A method of extracting an MAA, the method comprising:
a culture step of culturing a black yeast of the family Dothioraceae of the order Dothideales; and
an extraction step of extracting an MAA fraction from cultured fungal cells of the black yeast into a polar solvent;
wherein the survival rate of the fungal cells after the extraction step is not more than 20% compared to the fungal cells after the culture step.

13. The method according to claim 12, comprising, during or before the extraction step, exposing the cultured fungal cells and/or the culture liquid after the culture step to not less than 40°C.

14. The method according to claim 12, wherein the polar solvent is water.

15. The method according to any one of claims 12 to 14, wherein the black yeast is a black yeast belonging to the genus *Aureobasidium,* the genus *Hormonema (Sydowia),* or the genus *Hortaea.*

16. A method for producing a polar solvent extract of cultured fungal cells of a black yeast of the family Dothioraceae of the order Dothideales, the method comprising extracting an MAA by the method according to claim 5 or 12.

17. A medium for culturing a black yeast of the family Dothioraceae of the order Dothideales, the medium not substantially comprising ascorbic acid or a salt thereof, and/or peptone.

18. The medium according to claim 17, wherein the black yeast is a black yeast belonging to the genus *Aureobasidium,* the genus *Hormonema (Sydowia),* or the genus *Hortaea.*

19. A method of producing an MAA-containing composition, the method comprising a heating step of heating an MAA-containing composition.

20. The production method according to claim 19, wherein the heating step is carried out at a temperature of not less than 40°C.

21. The production method according to claim 19, further comprising a pressurization step of pressurizing the MAA-containing composition.

22. The production method according to claim 19, wherein the MAA-containing composition comprises a cultured fungal cell extract of a black yeast of the family Dothioraceae of the order Dothideales.

23. The production method according to claim 19, comprising:
a culture step of culturing a black yeast of the family Dothioraceae of the order Dothideales;
an extraction step of extracting an MAA fraction from cultured fungal cells of the black yeast; and
a step of including the MAA fraction into the composition;
before the heating step.
